# EUROPÄISCHE PATENTANMELDUNG

(11) **EP 3 659 489 A1**
(43) Veröffentlichungstag der Anmeldung: **03.06.2020**
(21) Anmeldenummer: 19207389.8
(22) Anmeldetag: 06.11.2019
(51) Int. Cl.: A61B 1/00, G02B 23/24

(54) **ENDOSKOP**

(30) Priorität: 30.11.2018 DE 102018130554
(71) Anmelder: Henke-Sass, Wolf GmbH, 78532 Tuttlingen (DE)
(72) Erfinder: MATTES, Andreas, 78589 Dürbheim (DE); PAULI, Sabine, 78606 Seitingen-Oberflacht (DE)
(74) Vertreter: Patentanwälte Geyer, Fehners & Partner mbB

(57) **Zusammenfassung**

Es wird ein Endoskop mit einem Endoskopschaft (2) und einem mit dem Endoskopschaft (2) verbundenen Hauptkörper (4), an dem ein erstes Einstellrad (12, 50) drehbar gelagert ist, bereitgestellt,
wobei zwischen einer ersten Dichtfläche des ersten Einstellrades (12, 50) oder eines mit dem ersten Einstellrad (12, 50) drehfest verbundenen ersten Bauteils und einer zweiten Dichtfläche des Hauptkörpers (4) oder eines mit dem Hauptkörper (4) drehfest verbundenen zweiten Bauteils eine Kunststoff-Dichtung angeordnet ist, wobei als Kunststoff ein Thermoplast verwendet ist.

## Beschreibung

Die vorliegende Erfindung betrifft ein Endoskop mit einem Endoskopschaft und einem mit dem Endoskopschaft verbundenen Hauptkörper, an dem ein erstes Einstellrad drehbar gelagert ist.

Solche drehbaren Einstellräder sind bei Endoskopen beispielsweise vorgesehen, um mittels der Drehstellung eine Fokussierlage einer Fokussieroptik einzustellen oder eine mit dem Drehrad drehfest verbundene Kamera zu drehen. Zum Drehen des Drehrades muss ein gewisses Mindestdrehmoment aufgebracht werden. Um ein Endoskop so herzustellen, dass ein gewünschtes Mindestdrehmoment eingestellt ist, werden sehr hohe Toleranzanforderungen an die entsprechenden Bauteile gestellt, was zu einer aufwendigen und teuren Herstellung führt.

Ausgehend hiervon ist es daher Aufgabe der vorliegenden Erfindung, ein Endoskop der eingangs genannten Art bereitzustellen, bei dem die genannten Schwierigkeiten möglichst vollständig behoben sind.

Die Erfindung ist im Anspruch 1 definiert. Vorteilhafte Ausgestaltungen sind in den abhängigen Ansprüchen angegeben.

Das erfindungsgemäße Endoskop kann einen Endoskopschaft und einen mit dem Endoskopschaft verbundenen Hauptkörper, an dem ein erstes Einstellrad drehbar gelagert ist, aufweisen. Zwischen einer ersten Dichtfläche des ersten Einstellrades oder eines mit dem ersten Einstellrad drehfest verbundenen ersten Bauteils und einer zweiten Dichtfläche des Hauptkörpers oder eines mit dem Hauptkörper drehfest verbundenen zweiten Bauteils kann eine Kunststoff-Dichtung angeordnet sein, wobei als Kunststoff ein Thermoplast verwendet ist.

Damit kann einerseits eine gute Dichtung erreicht werden. Andererseits kann ein unerwünschter Haftgleiteffekt (Stick-Slip-Effekt) vermieden werden, so dass eine bessere Einstellbarkeit des gewünschten Mindestdrehmoments möglich ist.

Die Kunststoff-Dichtung kann als ringförmige (z.B. kreisringförmige) Dichtung mit einem integrierten Vorspannelement ausgebildet sein. Insbesondere kann die ringförmige Dichtung als Nutring ausgebildet sein. Das Vorspannelement kann als Stahlfeder oder als Edelstahlfeder ausgebildet sein.

Als Kunststoff für die Kunststoff-Dichtung kann insbesondere eine Polyhalogenolefin und besonders bevorzugt Polytetrafluorethylen verwendet sein.

Bei dem erfindungsgmäßen Endoskop kann zwischen einer dritten Dichtfläche des ersten Einstellrades oder eines mit dem ersten Einstellrad drehfest verbundenen dritten Bauteils und einer vierten Dichtfläche des Hauptkörpers oder eines mit dem Hauptkörper drehfest verbundenen vierten Bauteils eine weitere Kunststoff-Dichtung angeordnet ist, wobei als Kunststoff ein Thermoplast verwendet ist.

Das dritte Bauteil kann dem ersten Bauteil entsprechen oder eine separates Bauteil sein. Das vierte Bauteil kann dem zweiten Bauteil entsprechen oder eine separates Bauteil sein.

Die weitere Kunststoff-Dichtung kann als ringförmige (z.B. kreisringförmige) Dichtung mit einem integrierten Vorspannelement ausgebildet sein. Insbesondere kann die ringförmige Dichtung als Nutring ausgebildet sein. Das Vorspannelement kann als Stahlfeder oder als Edelstahlfeder ausgebildet sein.

Als Kunststoff für die weitere Kunststoff-Dichtung kann insbesondere eine Polyhalogenolefin und besonders bevorzugt Polytetrafluorethylen verwendet sein.

Das erfindungsgemäße Endoskop kann somit ein, zwei oder auch mehrere Kunststoff-Dichtungen aufweisen, die aus einem Thermoplast und insbesondere aus Polyhalogenolefin und besonders bevorzugt aus Polytetrafluorethylen gebildet sind und die z.B. als ringförmige (z.B. kreisringförmige) Dichtung (z.B. als Nutring) mit einem integrierten Vorspannelement (z.B. eine Stahlfeder oder eine Edelstahlfeder) ausgebildet sind.

Beim erfindungsgemäßen Endoskop kann das erste Drehrad eine erste Anlagefläche aufweisen. Der Hautkörper kann eine zweite Anlagefläche aufweisen, an der die erste Anlagefläche anliegt, wobei sich die Anlageflächen beim Drehen des ersten Einstellrads gegeneinander bewegen. Es kann eine erste Einstelleinheit vorgesehen sein, mit der eine Druckkraft, mit der die erste Anlagefläche gegen die zweite Anlagefläche gedrückt ist, einstellbar ist, um ein Mindestdrehmoment einzustellen, das notwendig ist, um das erste Einstellrad zu drehen.

Da mittels der ersten Einstelleinheit die Druckkraft einstellbar ist, kann das gewünschte Mindestdrehmoment leicht bei z.B. der Montage des Endoskops eingestellt werden. Damit ist es möglich, höhere zulässige Toleranzen für die einzelnen Bauteile vorzugeben, die zwar zu größeren Schwankungen bei den Abmessungen der einzelnen Bauteile führen. Dies kann jedoch durch die einstellbare Druckkraft kompensiert werden, so dass letztendlich es sogar möglich ist, das gewünschte Mindestdrehmoment mit höherer Genauigkeit festzulegen.

Die erste Einstelleinheit ist insbesondere so ausgebildet, dass sie ein Benutzer im zusammengebauten Zustand des Endoskops nicht betätigen kann. In anderen Worten, sobald das Endoskop zusammengebaut ist, ist eine Verstellung durch einen Benutzer nicht möglich.

Die erste Einstelleinheit kann eine erste Feder aufweisen, deren Rückstellkraft einstellbar ist.

Die Verstellung der Rückstellkraft kann insbesondere durch eine Änderung der Länge der Feder bewirkt werden.

So kann die erste Einstelleinheit ein erstes Stellelement aufweisen, dessen axiale Position verstellbar ist, wobei ein Verstellen der axialen Position des ersten Stellelementes die Länge der ersten Feder und dadurch die Rückstellkraft der ersten Feder ändert.

Insbesondere kann das erste Stellelement über eine erste Schraubverbindung, die verschiedene Einschraubtiefen ermöglicht, mit dem Hauptkörper verbunden sein. Insbesondere kann eine Fixiereinheit vorgesehen sein, die eine eingestellte Einschraubtiefe fixiert.

Die erste Feder kann als Spiralfeder ausgebildet sein. Ferner kann die erste Feder aus Metall und insbesondere aus Edelstahl gebildet sein.

Die Anlageflächen können quer zu einer Längsrichtung des Endoskopschafts ausgerichtet sein und die Druckkraft kann parallel zur Längsrichtung wirken.

Eine der Anlageflächen kann eine Fläche eines Kunststoff-Gleitlagers sein. Das Kunststoff-Gleitlager kann insbesondere als Anlaufscheibe ausgebildet sein.

Das erste Einstellrad kann axial verschiebbar sein, um die Druckkraft einzustellen.

Die zweite Anlagefläche kann axial verschiebbar sein, um die Druckkraft einzustellen.

Das Endoskop kann einen Optikkanal aufweisen, in dem eine in Axialrichtung verschiebbare Optikeinheit angeordnet ist. Das erste Einstellrad kann so mit der Optikeinheit gekoppelt sein, dass bei Drehung des ersten Einstellrades die Optikeinheit axial bewegt wird. Die Kopplung kann beispielsweise über Magnete bewirkt sein.

Das erste Einstellrad kann einen Anschluss aufweisen, an dem eine Kamera oder ein Optikkoppler mechanisch drehfest befestigbar ist, so dass eine Drehung des ersten Einstellrades eine Drehung der Kamera oder des Optikkopplers bewirkt.

Das erfindungsgemäße Endoskop kann ein eine dritte Anlagefläche aufweisendes zweites Einstellrad, das am Hauptkörper drehbar gelagert ist, aufweisen, wobei der Hauptkörper eine vierte Anlagefläche umfasst, an dem die dritte Anlagefläche anliegt. Die dritte und vierte Anlagefläche bewegen sich beim Drehen des zweiten Einstellrades gegeneinander und es ist eine zweite Einstelleinheit vorgesehen, mit der die Druckkraft, mit der die dritte Anlagefläche gegen die vierte Anlagefläche gedrückt ist, einstellbar ist, um ein Mindestdrehmoment einzustellen, das notwendig ist, um das zweite Einstellrad zu drehen.

Das erfindungsgemäße Endoskop umfasst somit zwei Einstellräder, die voneinander unabhängig gedreht werden können und für die voneinander unabhängig das Mindestdrehmoment eingestellt werden kann, das notwendig ist, um ein Drehen zu bewirken.

Das zweite Einstellrad kann in gleicher Weise aus- und weitergebildet sein, wie das erste Einstellrad.

Insbesondere ist es möglich, dass das erste Einstellrad so mit der in einem Optikkanal angeordneten Optikeinheit gekoppelt ist, dass bei Drehung des ersten Einstellrades die Optikeinheit axial bewegt wird. Diese axiale Bewegung kann z.B. zur Fokussierung dienen. Das zweite Einstellrad kann einen Anschluss aufweisen, an dem eine Kamera oder ein Optikkoppler mechanisch drehfest befestigbar ist. Somit kann eine Drehung mittels des zweiten Einstellrades eine Drehung der Kamera oder des Optikkopplers bewirken.

Das Endoskop kann im Endoskopschaft ein Objektiv und eine Übertragungsoptik aufweisen. Mit dem Objektiv und der Übertragungsoptik kann ein vor dem distalen Ende des Endoskops befindliches Objekt abgebildet und zum proximalen Ende des Endoskops hin übertragen werden.

Das Endoskop kann als Geradeausblick-Endoskop (Blickrichtung entspricht der Längsachse des Endoskopschafts) oder als Schrägblick-Endoskop (Blickrichtung weist einen Winkel von ungleich 0° zur Längsachse des Endoskopschafts auf) ausgebildet sein. Der Endoskopschaft kann starr oder flexibel sein. Das Endoskop kann so ausgebildet sein, dass das Objektiv und die Übertragungsoptik gegenüber der Umgebung hermetisch dicht abgeschottet sind. Insbesondere kann das Endoskop autoklavierbar sein.

Das Endoskop kann als technisches Endoskop oder insbesondere als medizinisches Endoskop ausgebildet sein.

Zwischen einer ersten Dichtfläche des ersten Einstellrades oder eines mit dem ersten Einstellrad drehfest verbundenen ersten Bauteils und einer zweiten Dichtfläche des Hauptkörpers oder eines mit dem Hauptkörper drehfest verbundenen zweiten Bauteils kann eine Kunststoff-Dichtung angeordnet sind, wobei als Kunststoff ein Thermoplast verwendet ist.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbespiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:
- Fig. 1: eine schematische Darstellung eines erfindungsgemäßen Endoskops 1;
- Fig. 2: eine vergrößerte Darstellung des Hauptkörpers 4 des Endoskops 1 von Fig. 1;
- Fig. 3: ein Längsschnitt A-A durch den Hauptkörper 4 von Fig. 2;
- Fig. 4: eine perspektivische Explosionsdarstellung des Hauptkörpers 4, wobei ein proximales Abschlusselement 15 in Fig. 4 nicht dargestellt ist;
- Fig. 5: eine Explosionsdarstellung gemäß Fig. 4 in Seitenansicht;
- Fig. 6 und 7: eine Draufsicht und eine Schnittansicht C-C des Drehrades 12 des Optikanschlusses 5;
- Fig. 8 und 9: eine Draufsicht und eine Schnittansicht D-D des proximales Abschlusselementes 15 des Optikanschlusses 5;
- Fig. 10 und 11: eine Draufsicht und eine Schnittansicht E-E der Anschluss-Stellscheibe 28 und der Anschluss-Schraubbuchse 29;
- Fig. 12 und 13: eine Draufsicht und eine Schnittansicht F-F der Kunststoff-Abdeckung 35;
- Fig. 14: eine schematische Schnittansicht des Profils der ersten ringförmigen Dichtung 38;
- Fig. 15: eine Explosionsdarstellung eines Teils des Hauptkörpers zur Erläuterung der Einstellung des Mindestdrehmoments für das Fokussierrad 50;
- Fig. 16: die Explosionsdarstellung gemäß Fig. 15 in perspektivischer Darstellung;
- Fig. 17: die Schnittansicht B-B von Fig. 2 und
- Fig. 18 und 19: eine Schnittansicht G-G und eine Draufsicht des Fokussierrades 50 zusammen mit der Fokussiergleitbuchse 51.

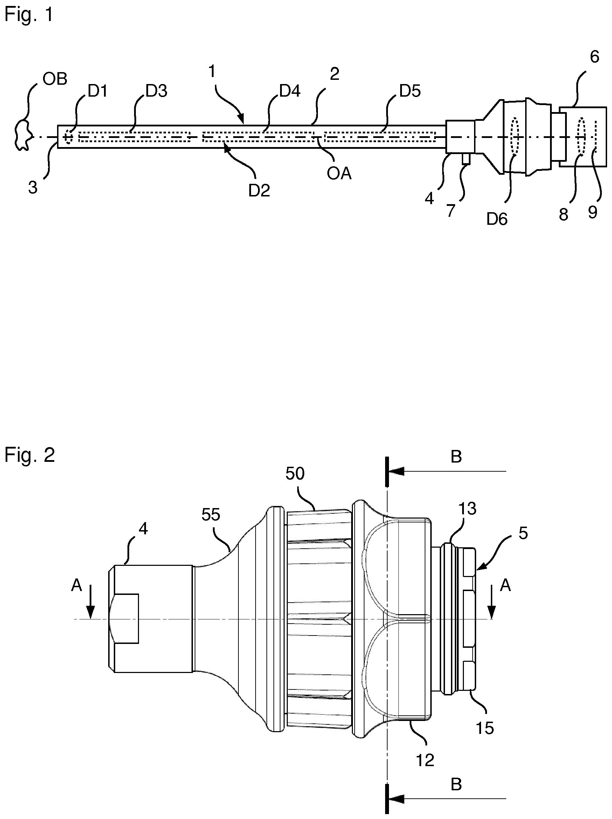

Bei dem in Fig. 1 und 2 gezeigten Ausführungsbeispiel umfasst das erfindungsgemäße Endoskop 1 einen Endoskopschaft 2 mit einem distalen Ende 3, das gleichzeitig das distale Ende des Endoskops 1 ist. Das dem distalen Ende 3 abgewandte Ende des Endoskopschaftes 2 ist mit einem Hauptkörper 4 des Endoskops 1 verbunden, der einen Optikanschluss 5 (der z.B. einen C-Mount-Anschluss 13 oder einen Bajonettanschluss umfassen kann) für eine optische Aufnahmevorrichtung 6 (beispielsweise eine Videokamera) oder eine optische Koppelvorrichtung aufweist. Ferner umfasst der Hauptkörper 4 einen Beleuchtungslichtanschluss 7. Die optische Aufnahmevorrichtung 6, die beispielsweise eine Optik 8 und einen CMOS-Sensor 9 enthält, ist lediglich in der Darstellung von Fig. 1 schematisch eingezeichnet und kann, muss aber nicht, Teil des erfindungsgemäßen Endoskops 1 sein.

Das Endoskop 1 kann im Endoskopschaft 2 an seinem distalen Ende 3 ein Objektiv D1 aufweisen, dem eine Übertragungsoptik D2 nachgeordnet ist, die beispielsweise drei Stablinsen D3, D4 und D5 umfassen kann. Im Hauptkörper 4 kann eine weitere Optik D6 angeordnet sein, die beispielsweise als Fokussieroptik D6 ausgebildet sein kann. In Fig. 1 ist ferner eine optische Achse OA der Optik D1 - D6 eingezeichnet, die parallel zur Längsrichtung des Schaftes 2 verläuft.

Somit kann in bekannter Art und Weise ein sich vor dem distalen Ende 3 befindliches Objekt OB mittels des Objektives D1, der Übertragungsoptik D2 und der Optik D6 zum proximalen Ende des Endoskops 1 hin abgebildet werden. Die Abbildung kann nach unendlich oder in eine Fokussierebene erfolgen, die innerhalb oder außerhalb des Endoskops 1 liegen kann. In dem in Fig. 1 gezeigten Ausführungsbeispiel ist die Abbildung so gewählt, dass mittels der Aufnahmevorrichtung 6 die gewünschte Abbildung auf dem CMOS-Sensor 9 erfolgen kann, wodurch eine digitale Bildaufnahme möglich ist.

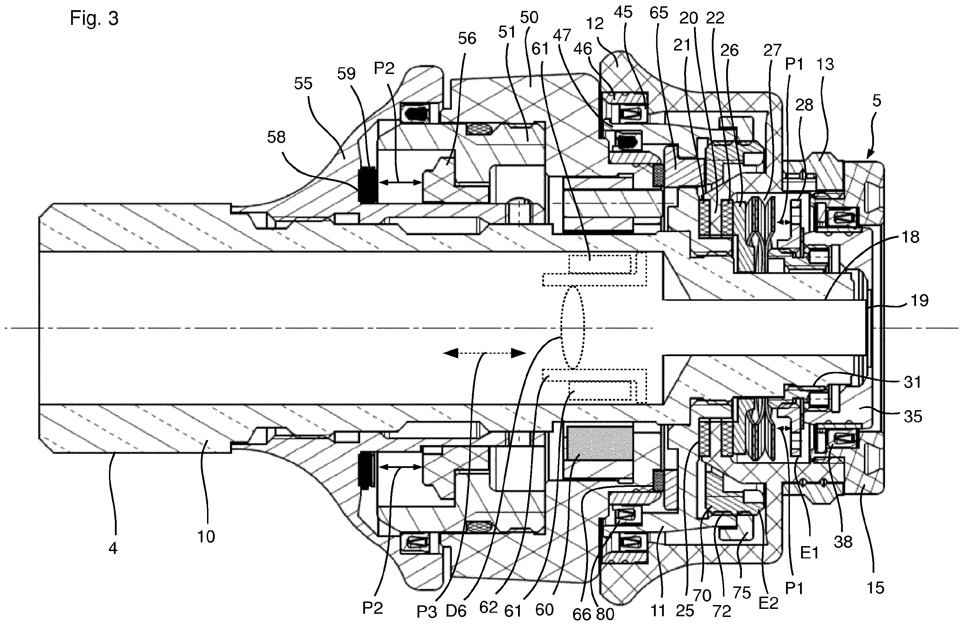

Wie insbesondere in den Fig. 3 ersichtlich ist, ist der Optikanschluss 5 gegenüber einem Grundkörper 10 des Hauptkörpers 4 drehbar gelagert, wobei das notwendige Drehmoment zum Drehen des Optikanschlusses 5 einstellbar ist. Diese Einstellung wird bevorzugt während der Montage des Endoskops 1 durchgeführt.

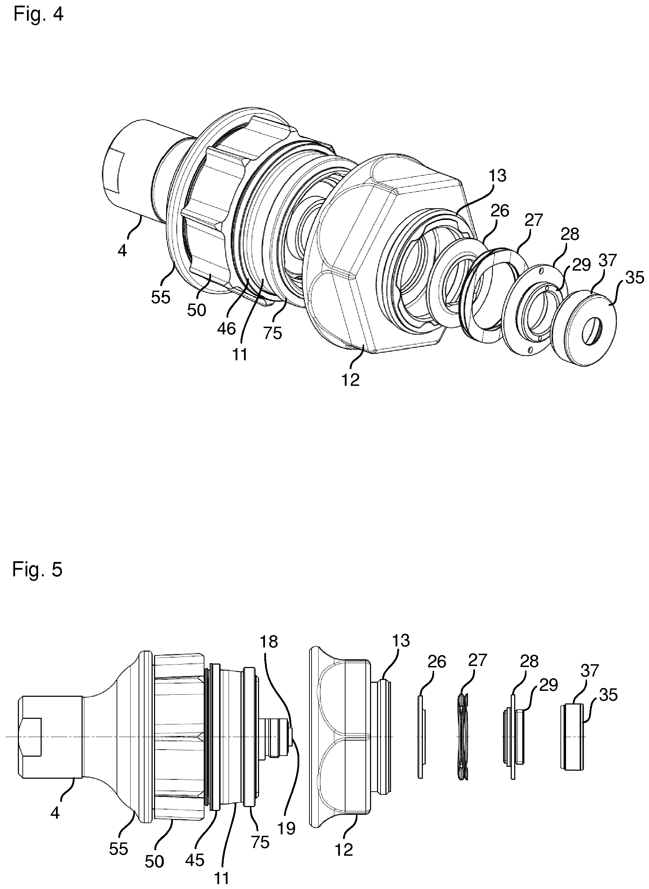

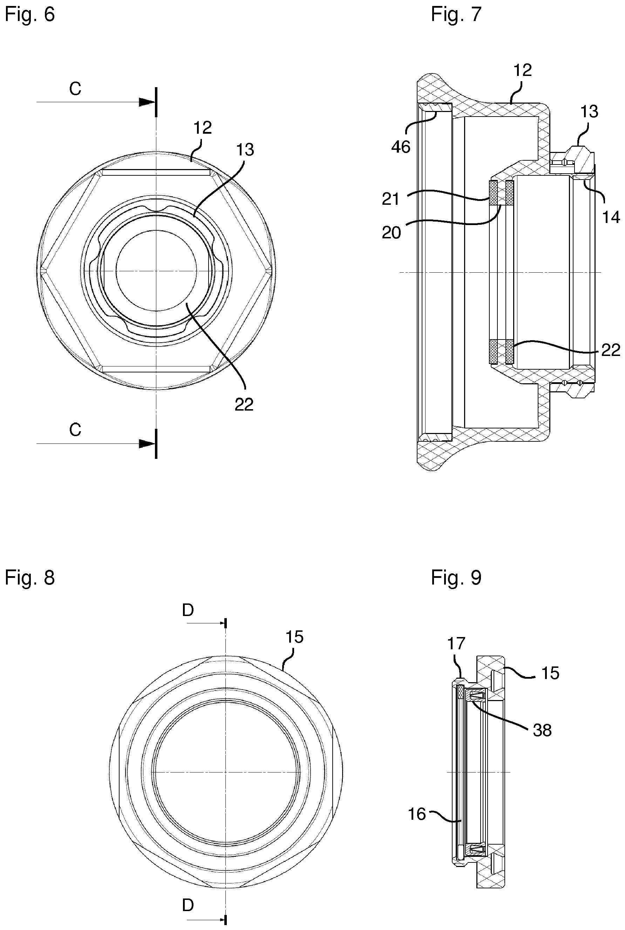

Dazu weist das Endoskop 1 ein drehfest mit dem Grundkörper 10 verbundenes Kopplerlager 11 (das auch in Fig. 4 und 5 dargestellt ist) auf, gegenüber dem ein Anschlussrad 12 drehbar gelagert ist. An seinem proximalen Ende weist das Anschlussrad 12 ein Aufnahmegewinde 13 (das als Außengewinde ausgebildet ist) auf, auf das die Aufnahmevorrichtung 6 aufgeschraubt werden kann. Ferner umfasst das Anschlussrad 12 an seinem proximalen Ende ein Innengewinde 14 (Fig. 7), in das ein proximales Abschlusselement 15 (Fig. 8 und 9) einschraubbar ist, das somit das proximale Ende des Endoskops 1 bildet. Durch das proximale Abschlusselement 15 kann Licht zur Durchführung von z.B. gewünschten Aufnahmen der endoskopischen Betrachtungen laufen. Dazu umfasst das Endoskop 1 einen Optikkanal 18, dessen proximales Ende 19 z.B. in Fig. 5 sichtbar ist. Das proximale Ende des Optikkanals 18 kann durch ein proximales Abschlussglas 16 (Fig. 3) hermetisch dicht verschlossen sein. Zum Einschrauben in das Innengewinde 14 weist das proximale Abschlusselement 15 ein entsprechendes Außengewinde 17 auf. Natürlich kann auch das distale Ende des Optikkanals 18 hermetisch dicht verschlossen sein, sodass das Objektiv D1, die Übertragungsoptik D2 und die weitere Optik D6 luftdicht gegenüber der Umgebung isoliert sind.

Das Anschlussrad 12 umfasst ferner einen inneren Ringabschnitt 20 (Fig. 7), an dessen beiden Seiten ein distales Anschluss-Gleitlager 21 und ein proximales Anschluss-Gleitlager 22 angeordnet sind. Die Gleitlager 21, 22 können als Kunststoff-Gleitlager und insbesondere als Polymergleitlager ausgebildet sein. Insbesondere können sie als Anlaufscheiben verwirklicht sein.

Im zusammengebauten Zustand liegt das distale Anschluss-Gleitlager 21 an einer ersten ringförmigen Anschlagfläche 25 des Kopplerlagers 11 an. Gegen das proximale Anschluss-Gleitlager 22 drückt eine Anschluss-Druckscheibe 26, die von einer Anschlussfeder 27 in Richtung zum proximalen Anschluss-Gleitlager 22 gedrückt wird. Dazu liegt die Anschlussfeder 27 mit ihrem anderen Ende an einer Anschluss-Stellscheibe 28 an, wie durch die Doppelpfeile P1 in Fig. 3 angedeutet ist. Von der Anschlussfeder 27 ist in Fig. 3 nur ein Teil dargestellt.

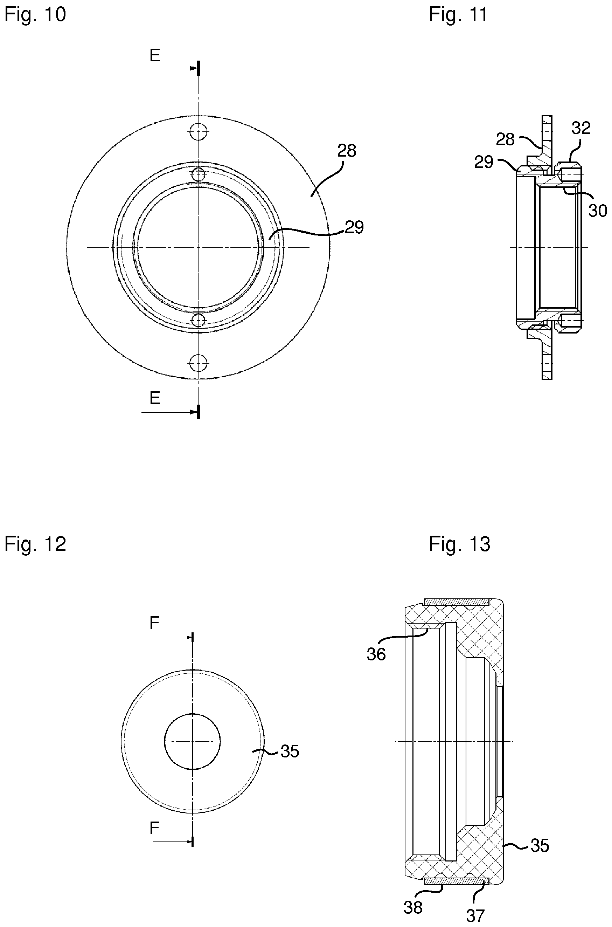

Die Anschluss-Stellscheibe 28 ist mit einer Anschluss-Schraubbuchse 29 (Fig. 10 und 11) verbunden, die ein Innengewinde 30 aufweist, mit dem sie auf ein entsprechendes Außengewinde 31 des Grundkörpers 10 aufschraubbar ist. Je nachdem, wie weit die Anschluss-Schraubbuchse 29 aufgeschraubt wird, wird der Abstand zwischen der Anschluss-Stellscheibe 28 und der Anschluss-Druckscheibe 26 und damit die Kraft, mit der die Anschlussfeder 27 gegen das proximale Anschluss-Gleitlager 22 drückt verändert und kann somit eingestellt werden. In dieser Art und Weise kann das gewünschte Drehmoment vorgegeben werden, das notwendig ist, um das Anschlussrad 12 zusammen mit dem Außengewinde 13 und dem aufgeschraubten proximalen Abschlusselement 15 zu drehen.

Die Anschluss-Stellscheibe 28, die Anschluss-Schraubbuchse 29, die Anschluss-Druckscheibe 26, und die Anschlussfeder 27 können als Einstelleinheit E1 bezeichnet werden, da mit ihnen die Druckkraft einstellbar ist, mit der die Anschluss-Druckscheibe 26 gegen das proximale Anschluss-Gleitlager 22 gedrückt wird.

Wie Fig. 11 zu entnehmen ist, weist die Anschluss-Schraubbuchse 29 ein Außengewinde 32 auf, auf das eine Kunststoffabdeckung 35 (Fig. 12 und 13) mit ihrem Innengewinde 36 aufschraubbar ist. Die Kunststoffabdeckung 35 weist an ihrer Außenseite eine Anschluss-Gleitbuchse 37 auf. Die Anschluss-Gleitbuchse 37 kann aus Stahl und insbesondere aus gehärtetem Stahl gebildet sein. Ihre Außenfläche 38 kann poliert sein.

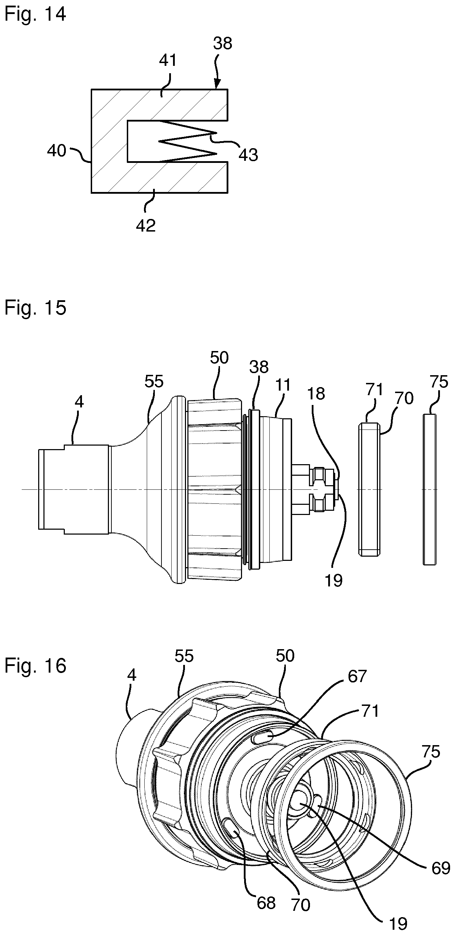

Zwischen der Anschluss-Gleitbuchse 37 und dem proximalen Abschlusselement 15 ist eine erste ringförmige Dichtung 38 angeordnet, die in Fig. 9 und Fig. 3 gezeigt ist. Die erste ringförmige Dichtung 38 kann insbesondere eine Kunststoffdichtung sein. Als Kunststoff kann insbesondere ein Thermoplast, wie z.B. ein Polyhalogenolefin verwendet werden. Besonders bevorzugt wird Polytetrafluorethylen (was auch häufig als PTFE oder Teflon bezeichnet wird) verwendet. Insbesondere ist die erste ringförmige Dichtung 38 als Kunststoff-Nutring mit einer integrierten Feder (z.B. Edelstahlfeder) als Vorspannelement ausgebildet. In diesem Fall kann der Kunststoff-Nutring 39 z.B. ein C-förmiges Profil 40 mit zwei freien Schenkeln 41 und 42 aufweisen, die durch eine Feder 43 nach außen vorgespannt werden (Fig. 14).

Die Verwendung von einem Thermoplast als Kunststoff und insbesondere von PTFE führt zu dem Vorteil, dass eine ausreichende Dichtigkeit bei gleichzeitig einer sehr geringen Reibung erreicht werden kann. Damit kann einerseits der unerwünschte Einfluss der Reibung einer solchen Dichtung auf das benötigte Drehmoment zum Drehen des Anschlussrades 12 verringert werden. Insbesondere wird der unerwünschte Haftgleiteffekt (Stick-Slip-Effekt) verringert bzw. vermieden, so dass das unerwünschte Ruckgleiten nicht mehr auftritt. Eine zweite ringförmige Dichtung 45 (Figuren 3-5 und 7) ist zwischen einem ringförmigen Innenabschnitt 46 des Anschlussrades 12 und einem ringförmigen Außenabschnitt 47 des Kopplerlagers 11 angeordnet. Die zweite ringförmige Dichtung 45 kann gleich ausgebildet sein wie die erste ringförmige Dichtung 38.

Ferner ist ein Fokussierrad 50 vorgesehen, das drehfest mit einer Fokussiergleitbuchse 51 verbunden ist (Figuren 3, 18 und 19). Auf dem Grundkörper 10 ist ein Rotorstopp 55 drehfest befestigt, der eine Lagerbuchse 56 so lagert, dass sie nur axial verschiebbar ist. Zwischen einem distalen Ende der Lagerbuchse 56 und einem Anschlag 58 des Rotorstopps 55 ist eine Fokussierfeder 59 angeordnet, die die Lagerbuchse 56 in Richtung zum proximalen Ende des Endoskops mit einer Kraft beaufschlagt, wie durch die Doppelpfeile P2 angedeutet ist. Von der Fokussierfeder 59 ist in Fig. 3 nur ein Teil dargestellt.

Der Abstand zwischen der Lagerbuchse 56 und dem Anschlag 58 des Rotorstopps 55 kann, wie nachfolgend im Detail beschrieben wird, verändert und eingestellt werden, um dadurch das gewünschte Drehmoment einzustellen, das notwendig ist, um das Fokussierrad 50 zu drehen. Das Fokussierrad 50 trägt einen oder mehrere Magnete 60, die mit Magneten 61 einer Optikeinheit 62 so zusammenwirken, dass ein Drehen des Magnets 60 bzw. der Magnete 60 dazu führt, dass diese Drehbewegung in eine axiale Bewegung (Doppelpfeil P3) der Optikeinheit 62 umgesetzt wird, wie in Fig. 3 schematisch dargestellt ist. Damit kann z.B. ein Fokussieren durchgeführt werden.

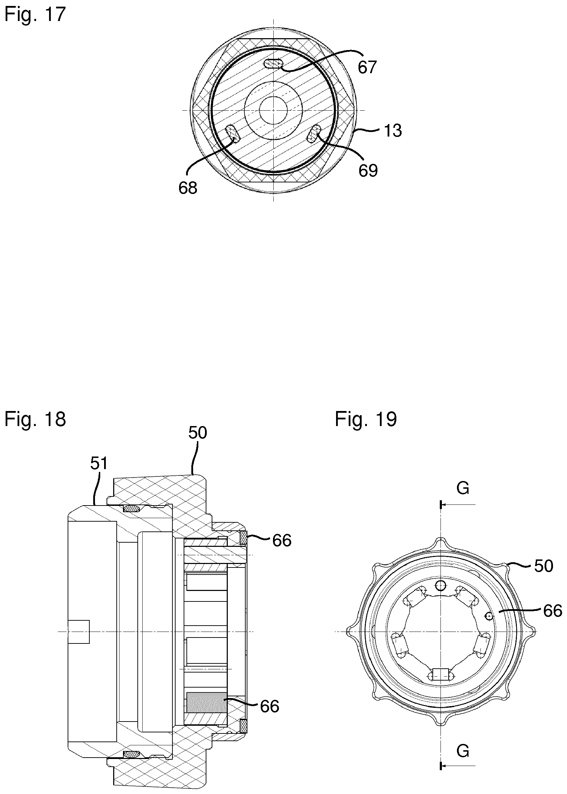

Es ist eine Fokussierdruckscheibe 65 in dem Kopplerlager 11 axial geführt und gleichzeitig gegen Rotation gesperrt, die gegen ein proximales Fokussiergleitlager 66 des Fokussierrades 50 drückt. Das proximale Fokussiergleitlager 66 kann gleich wie das distale Anschluss-Gleitlager 21 ausgebildet sein. Die Führung der Fokussierdruckscheibe 65 im Kopplerlager 11 kann z.B. durch drei sich axial erstreckende Vorsprünge 67, 68, 69 (Fig. 3, 16 und 17), die sich durch entsprechende Durchgangsöffnungen im Kopplerlager 11 erstrecken, realisiert sein. Die Vorsprünge 67 bis 69 können in Umfangsrichtung beispielsweise um 120° voneinander beabstandet sein, wie in Fig. 16 und 17 angedeutet ist.

Auf die Vorsprünge 67 bis 69 drückt eine in das Kopplerlager 11 eingeschraubt Fokussierstellscheibe 70. Dazu weist die Fokussierstellscheibe 70 ein Außengewinde 71 auf, das in ein entsprechendes Innengewinde 72 des Kopplerlagers 11 eingeschraubt ist. Je weiter die Fokussierstellschraube 70 eingeschraubt ist, desto weiter wird die Fokussierdruckschreibe 65 in Richtung zum distalen Ende des Endoskops 1 hin bewegt. Diese Bewegung führt zu einer axialen Verschiebung des Fokussierrades 50 mit der Fokussiergleitbuchse 51 und somit auch der Lagerbuchse 56, so dass der Abstand zwischen dem distalen Ende der Lagerbuchse 56 und dem Anschlag 58 des Rotorstopps 55 kleiner wird, wodurch die Fokussierfeder 59 stärker zusammengedrückt wird. Dies führt zu einer höheren Rückstellkraft und somit zu einem höheren Drehmoment, das nötig ist, um das Fokussierrad 50 zu drehen. Auf das Außengewinde 71 der Fokussierstellscheibe 70 ist eine Fokussiermutter 75 mit einem entsprechenden Innengewinde 76 aufgeschraubt, die die eingestellte Einschraubtiefe der Fokussierstellscheibe 70 fixiert.

Die Fokussierdruckscheibe 65, die Fokussierstellscheibe 70 kann zusammen mit der Fokussierfeder 59 als Einstelleinheit E2 bezeichnet werden, mit der die Kraft, mit der die Fokussierdruckscheibe gegen das proximale Fokussiergleitlager 66 drückt, eingestellt werden kann.

Zwischen dem Fokussierrad 50 und dem Kopplerlager 11 ist eine dritte ringförmige Dichtung 80 angeordnet. Ferner ist zwischen der Fokussiergleitbuchse 51 und dem Rotorstopp 55 eine vierte ringförmige Dichtung 81 angeordnet. Die dritte und vierte ringförmige Dichtung 80, 81 können in gleicher Weise wie die erste ringförmige Dichtung 38 ausgebildet sein.

Bei dem erfindungsgemäßen Endoskop 1 können somit das notwendige Drehmoment zum Drehen des Anschlussrades 12 und des Fokussierrades 50 voneinander unabhängig eingestellt werden. Durch die beschriebene Möglichkeit der individuellen Einstellung des zu überschreitenden Drehmomentes können in vorteilhafter Weise die zulässigen Toleranzen der einzelnen Bauteile größer sein im Vergleich zu bisher bekannten Lösungen, da die daraus resultierenden größeren Schwankungen in den Abmessungen der einzelnen Bauteile durch die beschriebene Einstellbarkeit der Drehmomente für das Anschlussrad 12 und das Fokussierrad 50 kompensiert werden können.

## Patentansprüche

1. Endoskop mit
einem Endoskopschaft (2) und
einem mit dem Endoskopschaft (2) verbundenen Hauptkörper (4), an dem ein erstes Einstellrad (12, 50) drehbar gelagert ist,
wobei zwischen einer ersten Dichtfläche des ersten Einstellrades (12, 50) oder eines mit dem ersten Einstellrad (12, 50) drehfest verbundenen ersten Bauteils und einer zweiten Dichtfläche des Hauptkörpers (4) oder eines mit dem Hauptkörper (4) drehfest verbundenen zweiten Bauteils eine Kunststoff-Dichtung angeordnet ist, wobei als Kunststoff ein Thermoplast verwendet ist.

2. Endoskop nach Anspruch 1, wobei die Kunststoff-Dichtung als ringförmige Dichtung mit einem integrierten Vorspannelement ausgebildet ist.

3. Endoskop nach Anspruch 2, wobei
die ringförmige Dichtung als Nutring ausgebildet ist.

4. Endoskop nach Anspruch 2 oder 3, wobei
das Vorspannelement als Stahlfeder ausgebildet ist.

5. Endoskop nach einem der obigen Ansprüche, bei dem
zwischen einer dritten Dichtfläche des ersten Einstellrades (12, 50) oder eines mit dem ersten Einstellrad (12, 50) drehfest verbundenen dritten Bauteils und einer vierten Dichtfläche des Hauptkörpers (4) oder eines mit dem Hauptkörper (4) drehfest verbundenen vierten Bauteils eine weitere Kunststoff-Dichtung angeordnet ist, wobei als Kunststoff ein Thermoplast verwendet ist.

6. Endoskop nach einem der obigen Ansprüche, bei dem
das erste Einstellrad (12, 50) eine erste Anlagefläche und der Hauptkörper (4) eine zweite Anlagefläche aufweist, an der die erste Anlagefläche anliegt, wobei sich die Anlageflächen beim Drehen des ersten Einstellrads (12, 50) gegeneinander bewegen, und wobei eine erste Einstelleinheit (E1, E2) vorgesehen ist, mit der eine Druckkraft, mit der die erste Anlagefläche gegen die zweite Anlagefläche gedrückt ist, einstellbar ist, um ein Mindestdrehmoment einzustellen, das notwendig ist, um das erste Einstellrad (12, 50) zu drehen.

7. Endoskop nach Anspruch 6, bei dem
die erste Einstelleinheit (E1, E2) eine erste Feder (27, 59) aufweist, deren Rückstellkraft verstellbar ist, um die Druckkraft einzustellen.

8. Endoskop nach Anspruch 6 oder 7, bei dem
die Anlageflächen quer zu einer Längsrichtung des Endoskopschafts (2) ausgerichtet sind und die Druckkraft parallel zur Längsrichtung wirkt.

9. Endoskop nach einem der Ansprüche 6 bis 8, bei dem
eine der Anlageflächen eine Fläche eines Kunststoff-Gleitlagers (22, 66) ist.

10. Endoskop nach Anspruch 9, bei dem
das Kunststoff-Gleitlager (22, 66) als Anlaufscheibe ausgebildet ist.
